Europäisches Patentamt

European Patent Office

Office européen des brevets

(11) Publication number: **0 507 256 A2**

## EUROPEAN PATENT APPLICATION

(21) Application number: **92105547.1**

(22) Date of filing: **31.03.92**

(51) Int. Cl.5: **A61K 37/64**

(30) Priority: **01.04.91 US 678779**

(43) Date of publication of application:
**07.10.92 Bulletin 92/41**

(84) Designated Contracting States:
**AT BE CH DE DK ES FR GB GR IT LI LU MC NL PT SE**

(71) Applicant: **Bristol-Myers Squibb Company**
**345 Park Avenue**
**New York, N.Y. 10154(US)**
Applicant: **SEATTLE BIOMEDICAL RESEARCH INSTITUTE**
**4 Nickerson Street**
**Seattle, Washington 98109(US)**

(72) Inventor: **Parsons, Marilyn**
**306 N. W. 113th Place**
**Seattle, Washington 98177(US)**
Inventor: **Ledbetter, Jeffrey A.**
**306 N. W. 113th Place**
**Seattle, Washington 98177(US)**
Inventor: **Schieven, Gary L.**
**4754 44th Avenue N.E.**
**Seattle, Washington 98105(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) Tyrosine phosphorylation in protozoa.

(57) A method for the treatment of microbial-induced disorders such as the protozoa-based diseases malaria and leishmania is described in which the phosphorylation of tyrosine residues in proteins is modulated. Specific phosphorylation of trypanosomal proteins, in T. brucei, is disclosed.

Tyrosine kinase and tyrosine phosphatase inhibitors are disclosed for administration to patients in the treatment method of the described invention.

## TECHNICAL FIELD OF THE INVENTION

The present invention is directed to the treatment of microbial-induced diseases such as malaria and leishmania using a tyrosine kinase or phosphatase inhibitor to regulate the phosphorylations of microbial proteins and, particularly, to inhibit tyrosine phosphorylation or dephosphorylation of proteins in trypanosomes.

## BACKGROUND OF THE INVENTION

Tyrosine phosphorylation is known to be a key regulator of cell proliferation and development in higher eukaryotes (Hunter, T. and Cooper, J.A. (1985) Ann. Rev. Biochem. 54:897-930). The modulation of the level of tyrosine phosphorylation has been found to play a major role in oncogenic transformation (Yarden, Y. and Ullrich, A. (1989) Ann. Rev. Biochem. 57:443-478; Karlund, J. (1985) Cell 41:707-717; Feldman, R.A., Lowy, D.R. and Vass, W.C. (1990) Oncogene Res. 5:187-197; Uehara, Y., Hori, M., Takeuchi, T. and Umezawa, H. (1985) Jpn. J. Cancer Res. 76:672-675). It has recently become evident that tyrosine kinases are important in growth and development in non-mammalian multicellular organisms such as Drosophila - (Hoffman, F.M. (1989) Curr. Top. Microbiol. Immunol. 147:1-29). When more divergent organisms such as Dictyostelium discoideum (Tan, J.L. and Spudich, J.A (1990) Mol. Cell. Biol. 10:3578-3583), Saccharomyces cerevisiae (Schieven, G., Thorner, J. and Martin, G.S. (1986) Science 231:390-393), and Schizosaccharomyces pombe (Gould, K.L. and Nurse, P. (1989) Nature 342:39-45) have been considered, all have been shown to possess tyrosine kinase genes or activities. The evolutionary distance between the protozoa, Trypanosoma brucei, and humans is more than twice the distance between humans and yeast, as calculated from analysis of the 16S ribosomal RNA subunits (Sogin, M.L., Gunderson, J.H., Elwood, H.I., Alonso, R.A and Peattie, D.A (1989) Science 243:75-77). At present, the role of tyrosine phosphorylation in protozoa such as T. brucei which diverged early in the eukaryotic lineage is not known.

T. brucei is an extracellular parasite with a highly regulated life cycle (Vickerman, K. (1985) Brit. Med. Bull. 41:105-114) and cell cycle (Woodward, R. and Gull, K. (1990) J. Cell Sci. 95:49-57). Trypanosomes move into $G_0$ at specific points, and then re-enter the cell cycle and move to the next stage of the life cycle. Non-cycling stages include stumpy bloodforms, and proventricular mesocyclic and metacyclic insect forms, while cycling forms include slender bloodforms, and procyclic and epimastigote insect forms (Vickerman, K. (1985) Brit. Med. Bull. 41:105-114).

Many highly passaged laboratory strains of T. brucei are continually cycling as slender forms. These monomorphic strains no longer differentiate to stumpy forms. Morphologically stumpy forms can be induced in such strains by treatment of the host with the drug difluoromethylornithine (DFMO) (Bacchi, C.J., Garofalo, J., Mockenhaupt, D., McCann, P.P., Diekema, K.A., Pegg, A.E., Nathan, H.C., Mullaney, E.A., Chunosoff, L., Sjoerdsma, A. and Hunter, S.H. (1983) Molec. Biochem. Parasitol. 7:209-225). The signals which regulate the transition from one stage of the life cycle to the next have not been defined in trypanosomes.

The present invention demonstrates that T. brucei possesses multiple proteins that react with an anti-phosphotyrosine antiserum and contain phosphotyrosine (p-tyr). Many of the tyrosine-phosphorylated proteins are detected in all available stages of the life cycle. However, a doublet at 40-42 kDa (ptp40 and ptp42) which is found in both stumpy bloodforms and procyclic forms is absent in slender bloodforms. Because slender bloodforms and procyclic forms are cycling, while stumpy forms are in $G_0$, this tyrosine phosphorylation appears to be stage-regulated, rather than cell cycle regulated. Treatment of procyclic forms with the tyrosine phosphatase inhibitor, orthovanadate, resulted in increased levels of tyrosine-phosyphorylated substrates, particularly the 40/42 kDa species.

## SUMMARY OF THE INVENTION

The present invention is directed to the treatment of microbial-induced disorders, such as malaria and leishmania, in patients. In the present invention a compound that regulates tyrosine phosphorylation is administered to a patient for a time period sufficient to inhibit the progression of the treated disorder. Compounds administered to the patient are tyrosine kinase inhibitors and/or tyrosine phosphatase inhibitors such as orthovanadate. Protozoa-induced diseases such as malaria, leishmania and trypanosomiasis are particularly amendable to the treatment method of the present invention in which phosphorylation of tyrosine residues in proteins is modulated.

Studies were conducted in the trypanosome, T. brucei, that demonstrated the formation of particular phosphoproteins having molecular weights of about 40 kDa and 42 kDa, respectively. Specific inhibitors of tyrosine kinase activity are further contemplated by the present invention.

## DESCRIPTION OF THE FIGURES

In the drawings:
FIGURE 1 illustrates the specificity of reactivity with anti-p-tyr antibodies. Lysate from the monomorphic trypanosome strain EATRO 164, clone IHRI 1, procyclic forms ($10^7$ cells) was loaded per lane on a 10% sodium dodecyl sulfate polyacrylamide gel electrophoresis (SDS-PAGE) gel. Proteins were transferred to polyvinylidene difluoride membranes, incubated with anti-p-tyr antibodies, and followed by treatment with $^{125}$I-protein A.

Panel A: 0.25 $\mu$g ml$^{-1}$ anti-p-tyr antibodies.

Panel B: 1 $\mu$g ml$^{-1}$ anti-p-tyr antibodies, plus 40 mM phenyl phosphate.

Molecular weight markers are indicated. Lanes 1 and 2 differ only in the concentration of orthovanadate used during cell processing.

FIGURE 2 illustrates immunoprecipitation and phosphoamino acid analysis.

Panel 2A: Western analysis of anti-p-tyr immunoprecipitates. Immunoreactive proteins were enriched by immunoprecipitation as described herein. Each lane was loaded with material corresponding to the same amount of cell lysate. Lanes: 1) total cell lysate; 2) lysate cleared of proteins reactive with anti-p-tyr; 3) eluate from anti-p-tyr immunoprecipitation.

Panel 2B: Two-dimensional phosphoamino acid analysis. Procyclic forms were metabolically labelled with [$^{32}$P] orthophosphate, and proteins reactive with the anti-p-tyr antiserum were enriched by immunoprecipitation as described above. The proteins were hydrolyzed and phosphoamino acids separated by two-dimensional electrophoresis. The radioactive spots comigrated exactly with phosphoamino acid standards revealed with ninhydrin: S, serine; T, threonine; Y, tyrosine.

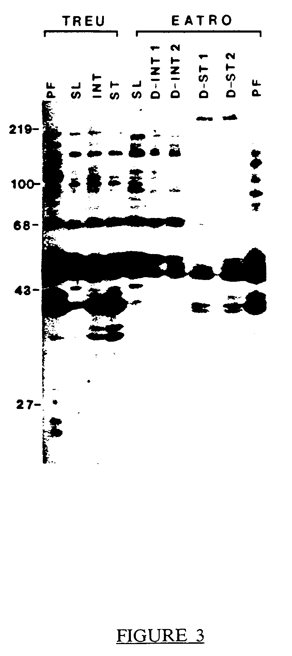

FIGURE 3 illustrates anti-p-tyr immunoblot analysis of different life cycle stages. Lysate of $10^7$ cells from each stage of the life cycle was loaded. TREU 667 is a pleiomorphic strain; EATRO 164 is a monomorphic strain. Independent samples produced by difluoromethylornithine (DFMO) treatment are indicated (D).

PF 100% mid-log phase procyclic culture forms.

SL Slender bloodforms (EATRO, 100% slender; TREU 75% slender).

INT Intermediate bloodforms (approximately 20% slender, 60% intermediate, 20% late intermediate-stumpy).

ST Stumpy bloodforms (>60% late intermediate-stumpy, the remainder intermediate).

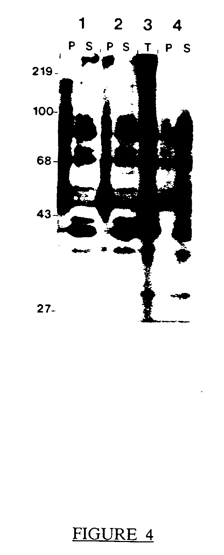

FIGURE 4 illustrates the solubility analysis of immunoreactive proteins. TREU 667 procyclic form cells were lysed in buffer containing various detergents as indicated. Lanes: 1) 1% NP-40 + 0.25% deoxycholate; 2) 1% NP-40; 3) 2% SDS; 4) freeze/thaw lysis. Solubilized proteins (S), insoluble pellets (P), total lysate (T).

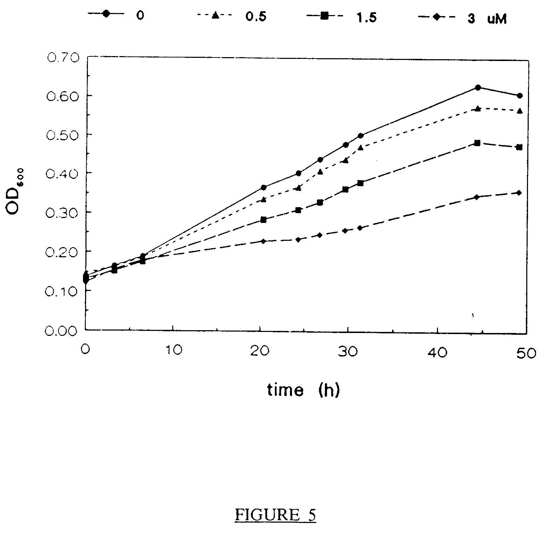

FIGURE 5 illustrates the effect of orthovanadate on cell growth. Mid-log phase TREU 667 procyclic form cells were incubated with various concentrations of orthovanadate. Growth of the parasites was monitored by measuring the optical density at 600 nm over ranges which were previously established to be linearly related to cell density.

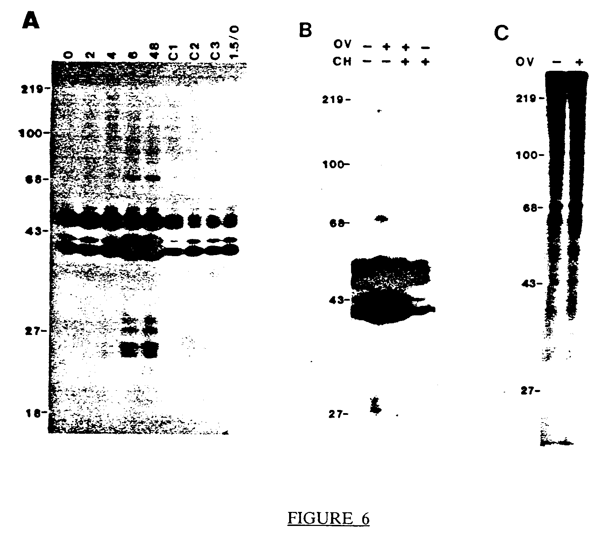

FIGURE 6 illustrates the effect of orthovanadate on protein phosphorylation and tyrosine phosphorylation in TREU 667 procyclic forms.

Panel 6A: Time course of orthovanadate treatment. Lysate from 3 x $10^6$ cells was loaded per lane. Time of incubation in 1.5 $\mu$M orthovanadate is indicated. 1.5/0 indicated cells grown in 1.5 $\mu$M orthovanadate for 24 hours, rinsed free of drug, and then grown for 24 hours. Control samples were mock-treated in medium lacking vanadate: C1), 0 h; C2) 48 h; C3) grown 24 h, rinsed, and then grown 2 h. The higher molecular weight bands are underexposed in order to more clearly reveal the induction of the lower molecular weight bands.

Panel 6B: Effect of cycloheximide on increased tyrosine phosphorylation mediated by orthovanadate. Cells were preincubated 15 min in cycloheximide (CH, 20 $\mu$g/ml) or in medium alone and then orthovanadate (OV) was added to 15 $\mu$M. The cells were incubated for 2 hours and then harvested for Western analysis using anti-p-tyr antibodies.

Panel 6C: SDS-PAGE analysis of proteins of cells metabolically labelled with [$^{32}$P]-orthophosphate.

Treatment with 1.5 $\mu$M ortho-vanadate (OV) was initiated one hour before the addition of [$^{32}$P]-orthophosphate for over-night labelling. The cells were rinsed three times and an aliquot equivalent to 2.3 x 10$^5$ cells was analyzed by SDS-PAGE and autoradiography.

## DESCRIPTION OF PREFERRED EMBODIMENTS

The present invention is directed to a method for treating a microbial-induced disorder by modulating the ability of the microbial organism to carryout protein phosphorylation.

The present invention is particularly directed to the control of tyrosine phosphorylation by protozoa, such as the trypanosome T. brucei. Tyrosine kinase inhibitors, such as genestein and tyrphostin and/or tyrosine phosphatase inhibitors, such as orthovanadate, are admininstered at a therapeutically effective dosage amount to a patient with a microbial infection for a time period sufficent to effectively modulate protein phosphorylation and proliferation by the infective organism. Administration of compounds to patients can be by any medically appropriate route such as by intravenous, oral, parenteral or suppository administration.

It is contemplated that the present invention provides an effective method for the treatment of microbial-induced diseases, and, particularly protozoan-induced diseases, such as malaria, leishmania and trypanosomiasis.

The present invention demonstrates that T. brucei, a protozoan parasite, possesses multiple proteins phosphorylated on tyrosine. In this species, as in higher eukaryotes, p-tyr appears to be a very rare phosphoamino acid. Phosphoamino acid analysis of metabolically-labelled procyclic forms grown in culture demonstrates that the tyrosine phosphorylation is performed by the parasite. It also confirms that the epitope detected in trypanosome lysates is p-tyr, and not some unusual phosphoserine or phosphothreonine derivative as has been observed in yeast using a different anti-p-tyr antiserum. The T. brucei proteins reacting with the anti-tyr antiserum, or proteins associated with them, also contain phosphoserine and phosphothreonine, as do most other tyrosine-phosphorylated proteins in other species (Kanner, S.B., Reynolds, A.B. and Parson, J.T. (1989) I. Immunol. Meth. 120:115-124). This raises the possibility of intricate interaction between various kinases and their substrates, suggesting the potential for complex regulation.

The present studies indicate that a subset of p-tyr containing proteins, including ptp40 and ptp42, are differentially regulated during the life cycle of the trypanosome. Ptp40 and ptp42 are absent in immunoblots of slender bloodforms, but intermediate and stumpy bloodforms show levels approaching those observed in procyclic forms. Since stumpy forms are non-dividing, while procyclic forms are dividing, the abundance of ptp40 and ptp42 appears to be correlated with the stage of the life cycle, rather than with quiescent or dividing states. It is intriguing that increases in ptp40 and ptp42 occur early in the differentiation of bloodform parasites. It is possible that phosphorylation of these substrates is important for controlling differentiation processes. The exact relationship of DFMO-induced stumpy forms to naturally occurring stumpy forms is not clear; however, they share a number of metabolic characteristics (Vickerman, K. (1965) Nature 208:762-766; Feagin, J.E., Jasmer, D.P. and Stuart, K. (1986) Molec. Biochem. Parasitol. 20:207-214). The present invention shows that these similarities do not extend to the level of tyrosine phosphorylation, suggesting that the DFMO-treated stumpy form is distinct from the naturally occurring stumpy form.

The existence of tyrosine phosphorylation in T. brucei implies the existence of tyrosine kinases. It is now important to identify and characterized these kinases. Unicellular organisms are generally thought not to possess receptor type tyrosine kinases, because they do not exist in a "cellular" environment where intercellular communication is vital (Yarden, Y. and Ullrich, A. (1989) Ann. Rev. Biochem. 57:443-478). However, parasites such as T. brucei exist in a cellular environment, and may have evolved mechanisms to sense the host status, allowing them to regulate their proliferation or differentiation. Evidence for extracellular factors affecting the growth and development of T. brucei has been obtained. The most clearcut evidence is the demonstration of an epidermal growth factor receptor on T. brucei (Hide, G., Gray, A., Harrison, C.M. and Tait, A. (1989) Molec. Biochem. Parasitol. 36:51-60). Although the molecular nature of the T. brucei receptor has not been described, in mammalian cells the epidermal growth factor receptor is a protein tyrosine kinase (Yarden, Y. and Ullrich, A. (1989) Ann. Rev. Biochem. 57:443-478). Other data indicate that serum factors and possibly parasite-derived factors affect the differentiative capacity of trypanosomes (Otigbuo, I.N. and Wee, P.T.K. (1987) Soc. Trop. Med. Hyg. 81:408-410; Seed, J.R. and Sechelski, J.B. (1989) J. Protozool. 36:572-577). By analogy to other eukaryotes the present inventors have determined that signal transduction in T. brucei ccould include either receptor or cytoplasmic tyrosine kinases.

The present inventors have further established that other microbial pathogens, specifically, Leishmania, Trypanosoma cruzi and Giardia lamblia, all possess proteins that react with anti-phosphotyrosine antiserum which indicates that these organisms contain phosphotyrosine. Giardia is a pathogen associated with intestinal illness. Leishmania is known to cause a broad range of diseases including skin ulcers, acute visceral diseases, and chronic mucosal diseases; leishmania is endemic in South and Central America, the Middle East, areas of Africa and the Near East. T. cruzi has been implicated as a major cause of chronic heart disease in the world.

In conjunction with tyrosine kinases, tyrosine phosphatases regulate tyrosine phosphorylation of physiological substrates (Hunter, T. (1989) Cell 58:1013-1016). The ability of orthovanadate, a phosphotyrosine phosphatase inhibitor, to increase the level of p-tyr containing proteins, demonstrates that such phosphatases also function in these ancient organisms. As in other systems (Karlund, J. (1985) Cell 41:707-717; Feldman, R.A., Lowy, D.R. and Vass, W.C. (1990) Oncogene Res. 5:187-197), orthovanadate specifically increases the level of tyrosine phosphorylation, but does not cause major alteration in overall protein phosphorylation (which is primarily phosphoserine and phosphothreonine). However, the effects of vanadate are complex as it is an inhibitor of other enzymes not normally thought to function in tyrosine phosphorylation pathways (Karlund, J. (1985) Cell 41:707-717; Kanik-Ennulat, C. and Neff, N. (1990) Molec. Cell. Biol. 10:898-909). The relative contribution of increased tyrosine phosphorylation to the inhibition of growth remains to be elucidated. Whatever the mechanism, vanadate treatment provides a simple method for increasing the tyrosine phosphorylation of specific proteins, in particular ptp40 and ptp42 kDa, for future biochemical analyses and immunoaffinity purification.

The presence of regulated, phosphotyrosine-containing proteins in this ancient species suggests that phosphotyrosine regulatory circuits may be important in the growth and development of T. brucei and other primitive eukaryotes. Thus the characterization of T. brocei protein tyrosine kinases, tyrosine phosphatases, and their inhibitors becomes important as a potential route for the development of anti-parasite agents.

Many inhibitors of mammalian tyrosine kinase have been investigated by the present inventors to determine if tyrosine phosphorylation is reduced in the tested organisms.

In the presence of tyrosine phosphatase inhibitor, orthovanadate, which blocked the breakdown of phosphotyrosine, the tyrosine kinase inhibitors genestin and tyrphostin decreased tyrosine phosphorylation in T. brucei cells.

The present invention is further described by the following Examples which are intended to be illustrative and not limiting.

EXAMPLE 1

Immunoblot Analysis of Protein Phosphorylation

Western immunoblot analysis provides a sensitive, specific method for detecting proteins phosphorylated on tyrosine. Lysates of trypanosome procyclic forms grown in culture for the presence of proteins reactive with an affinity-purified anti-p-tyr antiserum were analyzed.

Trypanosomes

Monomorphic strain EATRO 164, clone IHRI 1 (Stuart, K, Gobright, E. Jenni, L., Milhausen, M., Thomashow, L.S. and Agabian, N. (1984) Parasitol. 70:747-754), and pleiomorphic strain TREU 667 (Michelotti, E.F. and Hajduk, S.L. (1987) J. Biol. Chem. 262:927-932) were used for all studies. Bloodforms were grown in either irradiated or non-irradiated rats, harvested, and separated from the host blood cells by DEAE chromatography (Lanham, S.M. (1968) Nature 218:1273-1274). Intermediate and stumpy forms of EATRO 164 were induced in monomorphic strains by treatment of the host with 2% DFMO in buffered drinking water for 2 and 4 days, respectively. The populations were characterized as to their composition by morphological analysis following Giemsa staining. Procyclic culture forms were grown in medium SDM-70 containing 10% SeruMax (Sigma) and harvested in mid-log phase.

Immunoblot Analyses

Antisera were raised and immunoblots performed according to the procedure of Kamps and Sefton (Kamps, M.P. and Sefton, B.M. (1988) Oncogene 2:305-315). Briefly, rabbits were immunized with random copolymers of p-tyr, alanine, and glycine conjugated to keyhole limpet hemocyanin. Antibodies were adsorbed onto solid phase phosphotyramine. Following washing with buffers containing phosphoserine and phosphothreonine, the antibodies were eluted, with phenyl phosphate, and extensively dialyzed. This antiserum does not react with phosphoserine or phosphothreonine.

In preparation for immunoblotting, cells were rinsed free of serum, lysed in hot sodium dodecyl sulfate polyacrylamide gel electrophoreses (SDS-PAGE) sample buffer containing 50 $\mu$M orthovanadate, and immediately boiled for 5 minutes. Aliquots containing $3 \times 10^6$ to $10^7$ cells (15-50 $\mu$g protein) were loaded onto 10% SDS-PAGE gels. Following electrophoresis the proteins were electrophoretically transferred to polyvinylidene difluoride (PVDF) membranes. The blots were incubated with anti-p-tyr antibodies and bound antibodies were revealed by $^{125}$I-protein A.

For solubility analyses, procyclic form cells were lysed in lysis buffer (50 mM Tris HCl, 150 mM NaCl, 1 mM orthovanadate, 1 mM phenylmethylsulfonyl fluoride, pH 7.5) plus 2 mM EGTA, 50 $\mu$g ml$^{-1}$ leupeptin, and various detergents. The solublized proteins were separated from the pellet by centrifugation at 15000 x g for 5 minutes.

For phosphoamino acid analysis, 5 x 10$^8$ midlog phase TREU 667 procyclic forms were grown overnight in 5 ml phosphate-free Cunningham's medium (Cunningham, I. (1977) J. Protozool. 24325:19203-19329) containing 2 mCi [$^{32}$P]-orthophosphate. For SDS-PAGE analysis of total cellular phosphoproteins the incubation was scaled down 10-fold. The cells were washed twice in phosphate-free medium and lysed in 25 mM Tris-HCl containing 0.5% SDS and 1 mM dithiothreitol. The lysate was immediately placed in a boiling water bath for 5 minutes to inactivate phosphatases and proteases. The sample was diluted with 4 volumes of the lysis buffer (described above) supplemented with 0.5% aprotinin, 1 mM dithiothreitol, 1% NP-40, and 1% sodium cholate, and incubated for 10 min at 0 to 4°C to allow detergent exchange. The insoluble material was removed by centrifugation. 10 $\mu$g of purified anti-p-tyr was added and incubated at 4° for about 12 to 16 hours, and then the immune complexes were collected on protein-A Sepharose. The immunoprecipitates were washed four times with washing buffer (50 mM Tris HCl, 0.45 M NaCl, 0.5% NP-40, pH 8.3), and bound proteins were then released with SDS-PAGE sample buffer. Control experiments determined that cells incubated in phosphate-free medium still possessed significant levels of immunoreactive proteins. The same procedure was used to affinity purify immunoreactive proteins from unlabelled cells.

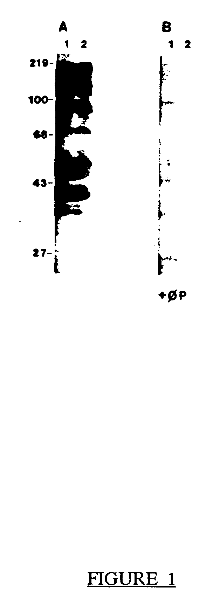

As shown in FIGURE 1A, many proteins ranging in molecular weight from 35 to 200 kDa were revealed. As shown in panel B, the reactivity is inhibited by 40 mM phenyl phosphate, demonstrating its specificity. Many of the bands observed, particularly those in the 40 to 55 kDa region, react strongly with monoclonal anti-p-tyr antibody 6G9, (Kanner, S.B., Reynolds, A.B., Vines, R.R. and Parson, J.T. (1990) Proc. Natl. Acad. Sci. USA 87:3328-3332) (not shown). Another monoclonal antibody, 5F11 (Kanner, S.B., Reynolds, A.B., Vines, R.R. and Parson, J.T. (1990) Proc. Natl. Acad. Sci. USA 87:3328-3332), reacted with other subsets of the proteins detected by the polyclonal antibodies.

EXAMPLE 2

Phosphoamino Acid Analysis

To confirm that the material detected by the antibody was truly p-tyr, phospho amino acid analyses on the immunoreactive proteins were performed.

Phosphoamino acid analysis followed the method of Cooper et al. (Cooper, J.A., Sefton, B.M. and Hunter, T. (1983) Meth. Enzymol. 99:387-402). Briefly, radiolabelled samples were spotted onto polyvinylidene difluoride paper, and hydrolysed for 1 h at 110°C in constant boiling HCl. After drying the samples were resuspended in first dimension buffer (pH 1.9) containing unlabelled phosphoserine, phosphothreonine, and p-tyr as internal standards. The samples were electrophoresed at 1000V for 45 min on 10 x 10 cm plates. The plates were dried, then they were wet in second dimension buffer (pH 3.5), and run at 1000V for 18 min. The standards were revealed with 0.2% ninhydrin, and the radiolabelled phosphoamino acids by autoradiography.

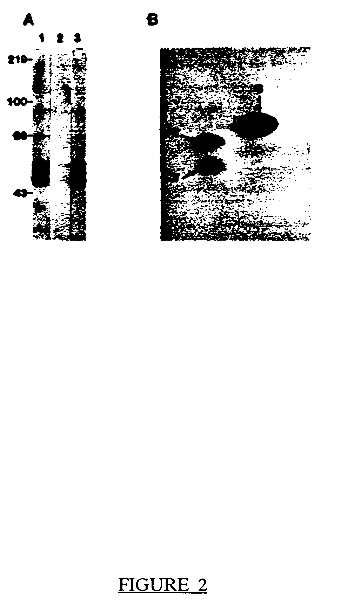

On two-dimensional electrophoresis of total $^{32}$P-labelled trypanosome lysates, the p-tyr spot was undetectable indicating that it represents a very small proportion of the total phosphamino acids (not shown). Therefore, the anti-p-tyr antibody plus protein A-Sepharose was used to enrich the material of interest. As shown by Western analysis (FIGURE 2A), this protocol removes the immunoreactive material from the cell lysate and the reactive proteins can be eluted from the beads by SDS-PAGE sample buffer. Procyclic form cells were labelled with [$^{32}$P]orthophosphate and the proteins reactive with anti-p-tyr antibody were isolated by immunoprecipitation. The proteins were eluted, hydrolyzed, and two-dimensional electrophoresis performed to identify the radiolabelled trypanosome phosphoamino acids. P-tyr was clearly present in these immunoprecipitates, as was phosphoserine and phosphothreonine (FIGURE 2B). In control immunoprecipitates using normal rabbit serum only a faint phosphoserine spot, and no p-tyr, was observed (not shown). These data confirm the presence of p-tyr in T. brucei procyclic forms and indicate that it is indeed a parasite product.

EXAMPLE 3

Tyrosine Phosphorylation During Trypanosome Life Cycle

The availability of parasites from different stages allows examination of tyrosine phosphorylation patterns as they relate to the life cycle. The present inventors compared procyclic forms with bloodforms from the monomorphic strain EATRO 164 and from the pleiomorphic (naturally differentiating) strain TREU 667. Lysates prepared from buffy coats collected at high parasitemias and the bloodform trypanosomes subsequently purified from them showed no consistent differences in p-tyr protein patterns (not shown), indicating that the purification of the parasite did not alter the phosphorylation of the major substrates. The most significant contaminant of the bloodform trypanosomes are red blood cells (<1%). When red blood cells equivalent to the typical contamination were analyzed, p-tyr containing proteins were not seen (not shown), indicating that the tyrosine-phosphorylated proteins are probably not derived from the host. This is supported by the observation that most of the tyrosine-phosphorylated proteins seen in bloodforms are the same as those seen in procyclic forms grown in the absence of host cells.

As shown in FIGURE 3, the different life cycle stages show different patterns on Western analysis. The most divergent samples are from DFMO-induced stumpy forms (D-ST1, D-ST2). Indeed, these cells exhibit an overall paucity of p-tyr containing proteins, with the exception of a unique high molecular weight protein. This contrasts with stumpy forms (ST) which arise from the normal differentiation of pleiomorphic cells. These data indicate that the DFMO-induced stumpy forms are not entirely homologous to the naturally occurring stumpy forms. Leaving the DFMO-treated cells aside, equivalent stages of the two strains show the similar patterns of tyrosine phosphorylation except for the largest protein. A 200 kDa protein is seen in TREU 667, while a 170 kDa protein is seen in EATRO 164. Whether these two proteins represent allelic forms of the same protein is as yet unknown.

The most striking differences in tyrosine phosphorylation during the life cycle are observed in the 40 kDa region of the gel. Two prominent species are seen in procyclic cell lysates which are designated ptp40 and ptp42. This doublet is only faintly visible in the slender bloodform lysates. As the pleiomorphic cells differentiate from slender to intermediate and then to stumpy, the intensity of these bands increases and approaches that seen in procyclic forms. Thus changes in the abundance or phosphorylation state of ptp40 and ptp42 occur early in the cycle of differentiation from slender forms to procyclic forms. As indicated above, DFMO-induced stumpy forms show lower tyrosine phosphorylation and only a relatively small increase in the intensity of these bands. There are other less dramatic differences between stages, with minor species specific to bloodforms (110 and 45 kDa) or procyclic forms (130 kDa).

EXAMPLE 4

Localization of Phosphorylated Proteins in T. Brucei

A number of p-tyr substrates and tyrosine kinases themselves have been described as being in the detergent-insoluble fraction of the cells (Zippel, R., Morello, L., Brambilla, R., Comoglio, PmM., Algerghina, L. and Sturani, E. (1989) Eur. J. Cell Biol. 50:428-434; Gold, M.R., Law, D.A., and DeFranco, A.L. (1990) Nature 345:810-813). T. brucei procyclic cells were separated into NP-40 soluble and insoluble fractions. Under these conditions, proteins associated with the cytoskeleton and other large polymeric structures remain insoluble. As shown in FIGURE 4, several proteins, including a prominent triplet around 50-55 kDa, and many of the higher molecular weight proteins, were found in the pellet fraction (P). Ptp40 and ptp42, as well as a few other proteins were found in the soluble supernatant (S). Sonication alone, however, released only a 80 kDa p-tyr protein (not shown).

EXAMPLE 5

Inhibition of Cell Growth

Effect Of Orthovanadate

The effect of orthovanadate, an inhibitor of p-tyr phosphatases (Swarup, G., Cohen, S. and Garbers, D.L. (1982) Biochem. Biophys. Res. Comm. 107:1104-1109), on the growth (FIGURE 5) and protein-tyrosine phosphorylation (FIGURE 6) of procyclic forms was investigated. Concentrations of orthovanadate approaching those used to cause phenotypic transformation of mammalian cells (30 $\mu$M) were toxic to trypanosomes. At 1.5 $\mu$M orthovanadate, the cells were nonmotile but continued to divide, albeit more slowly. After 6 hours in 1.5 mM orthovanadate the level of protein tyrosine phosphorylation of specific substrates, particularly ptp40 and ptp42, increased dramatically (FIGURE 6A). When the drug was removed, within 24 hours tyrosine phosphorylation returned to near control levels. At 15 $\mu$M orthovanadate, tyrosine phosphorylation was maximal within 2 hours (FIGURE 6B). The presence of cycloheximide at concentrations previously determined to arrest protein synthesis (unpublished results) does not prevent increased tyrosine phosphorylation. These results suggest that protein synthesis is not required for increased tyrosine phosphorylation, in accord with the concept that orthovanadate increases the level of tyrosine phosphorylation by stabilizing p-tyr. The specificity of the increase in tyrosine phosphorylation was studied by metabolically label-

ling cells with [$^{32}$P]orthophosphate in the presence of orthovanadate. The pattern of $^{32}$P-labelled proteins observed after SDS-PAGE was the same in control and treated cells (FIGURE 6C). Two-dimensional gel analyses confirmed this result (not shown).

The foregoing description and Examples are intended as illustrative of the present invention, but not as limiting. Numerous variations and modifications may be effected without departing from the true spirit and scope of the present invention.

**Claims**

1. The use of a compound that regulates tyrosine phosphorylation for preparing a pharmaceutical composition for treating a microbial-induced disorder in a patient.

2. The use according to Claim 1, wherein the disorder is a microbial infection, a protozoa-induced disease, or a giardia-induced disease.

3. The use according to Claim 2, wherein the protozoa are trypanosomes.

4. The use according to Claim 3, wherein the trypanosomes are T. brucei.

5. The use according to Claim 2, wherein the disease is malaria, a leishmania, a trypanosomiasis, or a giardia-induced disease.

6. The use according to Claim 1, wherein the compound is one that facilitates the phosphorylation of tyrosine residues.

7. The use according to Claim 6, wherein the phosphorylation comprises the formation of phosphoproteins having a molecular weight of about 40 to about 42 kilodaltons.

8. The use according to Claim 1, wherein the compound is a tyrosine kinase inhibitor, or a tyrosine phosphatase inhibitor.

9. The use according to Claim 8, wherein the inhibitor of tyrosine phosphorylation comprises a compound that effectively inhibits the enzymatic activity of a microbial tyrosine kinase.

10. The use according to Claim 9, wherein the compound is one that inhibits a protozoan tyrosine kinase.

11. The use according to Claim 10, wherein the protozoan is a trypanosome.

12. The use according to Claim 8 or 9, wherein the inhihitor is genestein, tryphostin, or orthovanadate.

FIGURE 1

FIGURE 2

FIGURE 3

FIGURE 4

FIGURE 5

FIGURE 6